# EUROPÄISCHE PATENTSCHRIFT

(11) **EP 0 535 316 B1**
(45) Veröffentlichungstag und Bekanntmachung des Hinweises auf die Patenterteilung: **24.05.1995**
(21) Anmeldenummer: 92111655.4
(22) Anmeldetag: 09.07.1992
(51) Int. Cl.: A61K 7/06, A61K 35/10

(54) **Antischuppenmittel**
Antidandruff compositions
Compositions antipelliculaires

(30) Priorität: 02.10.1991 DE 4132798
(43) Veröffentlichungstag der Anmeldung: 07.04.1993
(73) Patentinhaber: RÜTGERSWERKE AKTIENGESELLSCHAFT, 60326 Frankfurt (DE)
(72) Erfinder: Beilharz, Helmut, Dr., W-6905 Schriesheim (DE); Seubert, Bernhard, W-6803 Edingen-Neckarhausen 1 (DE); Riede, Urs, Prof. Dr., W-7800 Freiburg-St. Georgen (DE)

(56) Entgegenhaltungen:
- DE-A- 3 707 909
- SU-A- 1 327 888
- DATABASE WPIL Section Ch, Week 9214, Derwent Publications Ltd., London, GB;Class B, AN 92-110815

## Beschreibung

Die vorliegende Erfindung betrifft einen neuen Wirkstoff in Antischuppenpräparaten, vorzugsweise Antischuppenshampoos.

Kopfschuppen sind ein weitverbreitetes kosmetisches Problem und für ihre Bekämpfung sind bereits eine Vielzahl von Wirkstoffen vorgeschlagen und angewandt worden.

Das wohl beste und erfolgreichste Antischuppenmittel ist das aus US-A 3,236,733 bekannte Zink-Pyrithion, ein ökologisch und physiologisch unbedenklicher Wirkstoff, der in einfacher Weise dem Shampoo zugegeben wird, der sich bei der Haarwäsche auf der Kopfhaut verteilt und so fixiert, daß auch nach der Wäsche eine wirksame Menge auf der Kopfhaut verbleibt.

Allerdings haben Antischuppen-präparate oder -shampoos, die pyrithionhaltige Verbindungen enthalten, den Nachteil, daß sie die Kopfhaut anregen, stärker nachzufetten (vergl. Seifen-Ole-Fette-Wachse, 108 (1982), 471-475).

Es wird versucht, diese Nebenwirkung durch Reduzierung der Wirkstoffmenge sowie durch Zugabe weiterer Wirkstoffe, insbesondere von filmbildenden Fettethern, sog. Rückfettern zu minimieren.

Dennoch besteht ein Bedarf an einem Wirkstoff mit guter Antischuppenwirkung, der aber nicht die angesprochenen Nebenwirkungen hat und der ebenfalls einfach in ein Shampoo eingegeben werden kann, so daß die Antischuppenbehandlung mit der Haarwäsche erfolgen kann.

Die Lösung der Aufgabe erfolgt durch Verwendung niedermolekularer Alkalihuminate zur Herstellung von Antischuppenmitteln, insbesondere von Shampoos gemäß der Ansprüche 1 und 2, sowie durch niedermolekulare Alkalihuminate und ggf. wietere, kosmetisch oder medizinisch wirksamen Substanzen enthaltende Antischuppenmittel oder Haarshampoos gemäß den Ansprüchen 3 bis 6.

Es wurde gefunden, daß eine verdünnte, wäßrige Lösung niedermolekularer Ammonium- oder Alkalihuminate sowohl die Schuppenbildung als auch die Rückfettung der Haut unterdrückt, wenn sie auf die Haut, insbesondere auf die Kopfhaut aufgebracht wird. Außerdem werden die Keratinocyten pro inflammatorisch stimuliert.

Dabei kann diese wäßrige Lösung, die 0,1 bis 3 Gew.-% niedermolekularer Ammonium- oder Alkalihuminate und ggf. noch weitere, kosmetisch oder medizinisch wirksame Substanzen enthält, gezielt als Antischuppenmittel hergestellt und appliziert, d. h. auf Haare und Kopfhaut aufgebracht und nach einer Einwirkungszeit von 5 bis 15 min mit Wasser abgespült werden. Die niedermolekularen Alkalihuminate können aber auch in Mengen von 0,1 bis 3 Gew.-% in eine herkömmliche und an sich bekannte Haarshampoo-formulierung eingebracht werden und wirken im erfindungsgemäßen Sinn, wenn mit dem so hergestellten Shampoo die Haare und die Kopfhaut gewaschen werden.

In den erfindungsgemäßen Mitteln und Shampoos einsetzbar und wirksam sind sowohl natürliche niedermolekulare Alkalihuminate, wie sie aus DE-A 37 07 909 oder DE-A 37 36 623 bekannt sind, als auch synthetisch durch Oxidation von mehrwertigen Phenolen in schwach alkalischem wäßrigem Medium entsprechend DE-A 37 07 910 hergestellte niedermolekulare Alkalihuminate. Es sind dies dunkelbraune, wasserlösliche Produkte mit einem mittleren Molekulargewicht von 1.000, bei einem Streubereich von 300 bis 1.500.

Von diesen Wirkstoffen ist bekannt, daß sie als Heilmittel in der Wundheilung wirksam sind. Trotzdem ist es überraschend, daß diese Stoffe in geringer Konzentration bei nur kurzzeitiger Anwendung sowohl keratinolytisch als auch sebostatisch wirksam sind.

### Beispiel

Zu je 100 ml eines handelsüblichen Haarshampoos werden jeweils 50 ml einer 5 %igen wäßrigen Lösung eines niedermolekularen Alkalihuminats hergestellt
a. gemäß Beispiel 1 der DE-A 37 07 909
b. gemäß dem Beispiel der DE-A 37 07 910
c. gemäß Beispiel 2 der DE-A 37 36 623 zugegeben.

Verschiedene Probanden, die unter Kopfschuppen zu leiden hatten, verwandten jeweils eine der erhaltenen verdünnten Haarshampoos (jeweils 2 x 5 ml) bei der täglichen Wäsche von Haar und Kopfhaut. Die Ergebnisse waren in allen Fällen gleich: Nach einer Anwendungszeit von etwa 1 Woche war das Problem der Kopfschuppen nicht mehr akut. Auch nach mehrwöchigem Gebrauch der Shampoos war keine Erhöhung von Talgdrüsenaktivitäten auf der Kopfhaut zu beobachten. In einigen Fällen wurde subjektiv ein Rückgang der Haarfettung beobachtet.

## Patentansprüche

1. Verwendung niedermolekularer Ammonium- oder Alkalihuminate mit einem mittleren Molekulargewicht von 1000 bei einem Streubereich von 300 bis 1500 zur Herstellung eines Mittels zur Behandlung von Kopfschuppen.

2. Verwendung nach Anspruch 1, **dadurch gekennzeichnet,** daß die niedermolekularen Ammonium- oder Alkalihuminate einer handelsüblichem Shampoorezeptur in einer Menge von 0,1 bis 3 Gew.-%, bezogen auf das fertige Shampoo, zugesetzt werden.

3. Antischuppenmittel, **dadurch gekennzeichnet,** daß es 0,1 bis 3 Gew.-% niedermolekulare Ammonium- oder Alkalihuminate mit einem mittleren Molekulargewicht von 1000 bei einem Streubereich von 300 bis 1500 enthält.

4. Antischuppenmittel nach Anspruch 3, **dadurch gekennzeichnet,** daß es noch weitere, kosmetisch oder medizinisch wirksame Substanzen enthält.

5. Haarshampoo, **dadurch gekennzeichnet**, daß es 0,1 bis 3 Gew.-% niedermolekulare Ammonium- oder Alkalihuminate enthält.

6. Haarshampoo nach Anspruch 5, **dadurch gekennzeichnet**, daß es noch weitere, kosmetisch oder medizinisch wirksame Substanzen enthält.

## Claims

1. Use of low-molecular ammonium huminates or alkali huminates with an average molecular weight of 1000 with a scatter range of 300 to 1500 for the production of a remedy for the treatment of dandruff.

2. Use according to Claim 1, **characterized in that** the low-molecular ammonium huminates or alkali huminates are added to a commercial shampoo recipe in a quantity of 0.1 to 3 % by weight, relative to the ready-prepared shampoo.

3. All anti-dandruff remedy, **characterized in that** it contains 0.1 to 3 % by weight of low-molecular ammonium huminates or alkali huminates with an average molecular weight of 1000 with a scatter range of 300 to 1500.

4. All anti-dandruff remedy according to Claim 3, **characterized in that** it contains additional cosmetically or medicinally effective substances.

5. A hair shampoo, **characterized in that** it contains 0.1 to 3 % by weight of low-molecular ammonium huminates or alkali huminates.

6. A hair shampoo according to Claim 5, **characterized in that** it contains additional cosmetically or medicinally effective substances.

## Revendications

1. Utilisation d'humates d'ammonium ou de métaux alcalins de bas poids moléculaire ayant un poids moléculaire moyen de 1000 avec une plage de dispersion de 300 à 1500 pour la préparation d'un agent pour le traitement des pellicules de cheveux.

2. Utilisation selon la revendication 1, caractérisé en ce que les humates d'ammonium ou de métaux alcalins de bas poids moléculaire sont ajoutés à une formulation de shampooing du commerce en une quantité de 0,1 à 3% en poids par rapport au shampooing fini.

3. Agent antipelliculaire, caractérisé en ce qu'il contient 0,1 à 3% en poids d'humates d'ammonium ou de métaux alcalins ayant un poids moléculaire moyen de 1000 avec une plage de dispersion de 300 à 1500

4. Agent antipelliculaire selon la revendication 3, caractérisé en ce qu'il contient encore d'autres substances cosmétiquement ou médicalement efficaces.

5. Shampooing capillaire, caractérisé en ce qu'il contient 0,1 à 3% en poids d'humates d'ammonium ou de métaux alcalins de bas poids moléculaire.

6. Shampooing capillaire selon la revendication 5, caractérisé en ce qu'il contient encore d'autres substances cosmétiquement ou médicalement efficaces.
